# EUROPÄISCHE PATENTSCHRIFT

(11) **EP 3 501 466 B1**
(45) Veröffentlichungstag und Bekanntmachung des Hinweises auf die Patenterteilung: **26.07.2023**
(21) Anmeldenummer: 18213651.5
(22) Anmeldetag: 18.12.2018
(51) Int. Cl.: A61F 13/02, A61F 13/00, A61L 15/42, A61L 15/60, B26D 7/27, B26D 3/28, B26F 1/38

(54) **VERFAHREN ZUR BEARBEITUNG EINES SUBSTRATS**
METHOD FOR PROCESSING A SUBSTRATE
PROCÉDÉ DE TRAITEMENT D'UN SUBSTRAT

(30) Priorität: 21.12.2017 DE 102017131014
(43) Veröffentlichungstag der Anmeldung: 26.06.2019
(73) Patentinhaber: Paul Hartmann AG, 89522 Heidenheim (DE)
(72) Erfinder: Frühe, Thomas, 89518 Heidenheim (DE); Bernd, Frank, 89555 Steinheim (DE); Knecht, Theresia, 73433 Aalen (DE); Sprick, Ralf, 89564 Nattheim (DE)
(74) Vertreter: DREISS Patentanwälte PartG mbB

(56) Entgegenhaltungen:
- DE-A1- 2 438 486
- DE-A1- 3 623 738
- DE-A1- 10 249 874
- DE-A1-102014 212 374
- DE-T2- 60 026 343

## Beschreibung

Die Erfindung betrifft ein Verfahren zur Bearbeitung eines Substrats, insbesondere eines für medizinische Zwecke geeigneten Laminats.

Ein Substrat ist im vorliegenden Sinne ein flächiges und Material mit einer ersten Außenseite und einer zweiten Außenseite, wobei das Substrat mindestens eine poröse Lage umfasst. Das Substrat kann auch aus dieser porösen Lage, bspw. einem Polymerschaumstoff, bestehen.

Das Substrat kann ein Laminat, insbesondere ein für medizinische Zwecke geeignetes Laminat sein. Ein Laminat, das für medizinische Zwecke geeignet ist, ist im vorliegenden Sinne ein flächiges und mehrschichtiges Material mit einer ersten Außenseite und einer zweiten Außenseite, wobei das Laminat mindestens eine poröse Lage sowie eine Wundkontakt-Lage, die sich von der porösen Lage unterscheidet, aufweist. Unter der Bezeichnung "für medizinische Zwecke geeignet" wird im vorliegenden Zusammenhang verstanden, dass das Laminat ausschließlich physiologisch verträgliche Materialien enthält, die für eine Wundbehandlung geeignet sind.

Insbesondere kann das erfindungsgemäße Verfahren genutzt werden, um Wundauflagen herzustellen, insbesondere Wundauflagen, die geeignet sind, um zur feuchten Wundbehandlung eingesetzt werden können.

Bei der Wundbehandlung besteht ein Bedürfnis nach Wundauflagen, welche die Heilung von Wunden beschleunigen und den natürlichen Wundheilungsprozess unterstützen. Wundauflagen sollen dabei häufig unterschiedliche Eigenschaften in Kombination aufweisen.

Die Heilung von Hautwunden beruht auf der Fähigkeit der Haut Epithel sowie Binde- und Stützgewebe zu regenerieren. Die Regeneration selbst ist durch ein komplexes Geschehen ineinander übergreifender Zellaktivitäten gekennzeichnet, die den Heilungsprozess schrittweise vorantreiben.

Bei der Heilung von Hautwunden werden häufig drei wesentliche Heilungsphasen einer Wunde unterschieden, die inflammatorische oder exsudative Phase zur Blutstillung und Wundreinigung (Phase 1, Reinigungsphase), die proliferative Phase zum Aufbau von Granulationsgewebe (Phase 2, Granulationsphase) und die Differenzierungsphase zur Epithelisierung und Narbenbildung (Phase 3, Epithelisierungs-phase) .

Zur Unterstützung der einzelnen Wundheilungsphasen sind verschiedene Wundauflagen in der Literatur beschrieben.

In WO 02/ 38 097 A1, WO 02/ 47 761 A1, WO 03/ 011 352 A1, WO 03/ 086 255 A1, WO 2004/ 052 415 A1 oder EP 1 658 865 A1 werden Wundauflagen beschrieben, die ein Hydrogel und einen Polymerschaum umfassen. In WO 2010/000 450 des Anmelders Paul Hartmann AG, ist gleichfalls eine mehrschichtige bzw. mehrlagige Wundauflage mit einer Wundkontaktschicht als erster Schicht und mindestens einer zweiten Schicht als absorbierende Schicht, die einen hydrophilen Polyurethanschaum umfasst, beschrieben. Die Wundkontaktschicht kann ein Hydrogel auf Basis eines Polyharnstoff/Polyurethan-Copolymers sein.

Wundauflagen zur Behandlung chronischer Wunden werden typischerweise derart appliziert, dass der Rand der Wundauflage auf der die Wunde umgebenden, gesunden Haut zu liegen kommt. Beispielsweise werden Schaumwundauflagen, welche in Verbindung mit der Kompressionstherapie eingesetzt werden, auf diese Art appliziert. Die Wundauflage wird den Wundrand überlappend appliziert und mittels einer unter Druck gewickelten Binde fixiert. Durch die Auflage des Randes der Wundauflage auf der Haut kann sich für die in der Umgebung der Wunde vorhandene Haut eine erhebliche Belastung ergeben.

Wundauflagen mit abgeflachten Rändern können eingesetzt werden, um diese Belastungen zu mindern.

Schaumstoff-Wundauflagen, welche abgeflachte Ränder aufweisen, können im einfachsten Fall durch ein Beschneiden des Randes oder durch Gießen des noch nicht ausgehärteten Schaumes in eine entsprechend ausgestaltete Gießform erhalten werden, wie in WO91/01706 erwähnt.

Eine Möglichkeit zur Herstellung von Wundauflagen, welche abgeflachte Ränder aufweisen, ist in EP 1 931 289 B1 beschrieben. Demnach können abgeflachte Ränder erhalten werden, indem ein nur teilweise ausgehärteter Schaum randständig zusammengepresst wird. Der im Randbereich vorhandene Schaum weißt dann im Vergleich zum zentralen Bereich der Wundauflage eine erhöhte Dichte auf.

Die Möglichkeiten zur Bearbeitung von Wundauflagen, insbesondere zum Vorsehen von abgeflachten Rändern, sind eingeschränkt, wenn auf einer beispielsweise als Polymerschaumstofflage ausgebildeten porösen Lage zusätzlich eine oder mehrere Wundkontakt-Lagen, bspw. Hydrogel-Schichten, vorhanden sind. Dies kann bspw. zu Schwierigkeiten bei der Schaffung von abgeflachten Rändern führen.

Beim Einsatz thermischer Schneideverfahren kann das Hydrogel einen Teil der eingestrahlten Energie absorbieren und den Schneidevorgang behindern. Verfahren, die auf noch nicht vollständig ausgehärteten Polymer-Mischungen beruhen, können im Zusammenhang mit Gel-Schaumstoff-Laminaten überhaupt nicht eingesetzt werden.

Die beschriebenen Wundauflagen können aus Substraten im erfindungsgemäßen Sinne herstellbar sein. Es können auch Teile derartiger Wundauflagen durch Substrate, die mit dem erfindungsgemäßen Verfahren bearbeitet werden, gebildet werden.

DE 24 38 486 A1 offenbart ein Verfahren zum mustermäßigen Aufbringen eines Kunststoffpulvers auf ein Flachmaterial. DE 36 23 738 A1 offenbart ein Verfahren zum stellenweisen Beschichten oder Bedrucken von textilen Warenbahnen.

Aufgabe der vorliegenden Erfindung ist es, ein Verfahren bereitzustellen zur Bearbeitung, insbesondere Oberflächenkonturierung, eines flächigen Substrats, das eine poröse-Lage umfasst.

Diese Aufgabe wird erfindungsgemäß durch ein Verfahren zur Bearbeitung eines flächigen Substrats gemäß Anspruch 1 gelöst.

Das Verfahren zur Bearbeitung eines, vorzugsweise für medizinische Zwecke geeigneten, flächigen Substrats, das eine poröse-Lage umfasst, insbesondere die wiederum eine Schaumstoff-Lage oder ein Textil, insbesondere ein Fasergelege und/oder ein Fasergewebe, umfasst, umfasst die Schritte:
Schritt i) Bereitstellen des Substrats, wobei das Substrat eine erste Außenseite und eine zweite Außenseite aufweist.
Schritt ii) Auflegen des Substrats auf eine Arbeitsfläche, wobei die erste Außenseite die Arbeitsfläche nach dem Auflegen flächig kontaktiert. Das Auflegen des Substrats im vorliegenden Sinne ist dabei als flächiges in Kontakt bringen zu verstehen.
Schritt iii) Aufbringen einer Schablone, die eine Kontaktseite und eine Oberseite aufweist, vorzugsweise wobei die Schablone mindestens einen Durchbruch aufweist, wobei die Kontaktseite der Schablone beim Aufbringen auf die der Arbeitsfläche abgewandte zweite Außenseite des Substrats aufgebracht wird, wobei die Schablone einen bedeckten Teilbereich des Substrats abdeckt und ein freier Teilbereich des Substrats von der Schablone unbedeckt ist. Die Schablone kann einstückig ausgeführt sein. Der Durchbruch kann bspw. als Loch in der Schablone ausgeführt sein. Im Sinne der Erfindung ist auch die Verwendung von mehrteiligen Schablonen. Die Schablone kann bspw. mehrere Schablonenabschnitte bzw. Schablonenteile umfassen. Ein Durchbruch kann bspw. ein Loch in einem Schablonenteil sein oder ein Durchbruch kann durch entsprechendes Auflegen der Schablonenabschnitte bzw. Schablonenteile zwischen den Schablonenabschnitten gebildet sein. Ein Durchbruch ist vorzugsweise umfänglich geschlossen.
Schritt iv) Anpressen der Schablone in Richtung der Arbeitsfläche mittels einer direkt auf die Schablone wirkenden magnetischen Kraft, wobei das Substrat durch das Anpressen der Schablone in seiner Position auf der Arbeitsfläche gehalten, vorzugsweise und in seiner Erstreckung von der ersten Außenseite zur zweiten Außenseite komprimiert, wird, wobei in oder unter der Arbeitsfläche ein Permanentmagnet und/oder ein Elektromagnet angeordnet ist bzw. sind, so dass mittels des Permanentmagneten und/oder des Elektromagneten die zur Arbeitsfläche hin orientierte magnetische Kraft auf die Schablone ausgeübt werden kann. Durch das Anpressen der Schablone mittels magnetischer Kraft wird das Laminat gewissermaßen zwischen der Schablone und der Arbeitsfläche verklemmt. Die Schablone umfasst hierzu ferromagnetisches Material.
Schritt v) Bearbeiten wenigstens eines Teils des freien Teilbereichs des Substrats, vorzugsweise wobei das Bearbeiten ein Abtrennen eines Teils des freien Teilbereichs des Substrats, der über die Oberseite der Schablone hinausragt, umfasst und/oder wobei das Bearbeiten ein Aufbringen einer Substanz auf den freien Teilbereich des Substrats umfasst. Bspw. kann die Bearbeitung das Abtrennen des Teils des Substrats, der an den Seiten der Schablone über deren Oberseite hinaussteht, umfassen. Der Teil des Substrats, der abgetrennt wird, kann alternativ oder zusätzlich auch durch einen Durchbruch der Schablone über deren Oberseite hinausstehen. Bei der Bearbeitung des Substrats kann zusätzlich oder alternativ auch eine Substanz beispielsweise auf den freien Teilbereich aufgesprüht werden. Bei der Bearbeitung des Substrats kann zusätzlich oder alternativ auch der freie Teilbereich einer Strahlung ausgesetzt werden.
Vorzugsweise Schritt vi) Zuschneiden der Ränder des Substrats und/oder Aufteilen des Substrats, um mehrere Teilstücke bzw. Teilsubstrate zu erhalten, wobei das Zuschneiden und/oder Aufteilen insbesondere durch einen im Wesentlichen vertikal zu der Erstreckungsebene des Substrats geführten Schnitt erfolgt. Insbesondere kann das Substrat in diesem Schritt in einzelne Teilsubstrate, die beispielsweise gleich dimensioniert sein können, aufgeteilt werden. Der Schritt vi) kann auch mehrfach erfolgen. Insbesondere ist möglich, dass der Schritt vi) mehrfach und in verschiedenen Richtungen erfolgt. Beispielsweise kann der Schritt vi) eine Aufteilung in einer Längsrichtung und nachfolgend eine Aufteilung in einer Querrichtung umfassen.
Vorzugsweise Schritt vii) Aufbringen einer flächigen Materialschicht auf die erste Außenseite und/oder die zweite Außenseite des Substrats oder eines der Teilstücke.
Vorzugsweise Schritt viii) Sterilisieren und/oder Verpacken des Substrats oder eines Teilstücks des Substrats.

Vorzugsweise erfolgen zwei, vorzugsweise mehrere, vorzugsweise sämtliche Schritte i) bis viii) in der eben genannten Reihenfolge.

Das Verfahren kann weiterhin umfassen, Beaufschlagen und/oder Imprägnieren des Substrats mit einer, vorzugsweise wundheilungsfördernden, Substanz oder Zusammensetzung. Die Verwendung einer wundheilungsfördernden Substanz eignet sich besonders bei der Herstellung von Wundauflagen.

Denkbar ist jedoch auch beispielsweise die Beaufschlagung des Substrats mit einer farbgebenden Substanz. Die Substanz kann bspw. aufgesprüht werden.

Die Schablone kann einstückig, vorzugsweise mit einem Durchbruch, ausgebildet sein oder mehrstückig ausgebildet sein und mehrere Schablonenteile bzw. Schablonenabschnitte umfassen, vorzugsweise wobei die Schablonenabschnitte derart auf das Substrat aufgebracht sind, dass zwischen den Schablonenabschnitten ein Durchbruch gebildet ist. Die Schablone bzw. Schablonenabschnitte kann/können starr oder flexibel elastisch ausgebildet sein.

In oder unter der Arbeitsfläche ist ein Permanentmagnet und/oder ein Elektromagnet angeordnet, so dass mittels des Permanentmagneten und/oder des Elektromagneten eine zur Arbeitsfläche hin orientierte magnetische Kraft auf die Schablone, die hierzu ein ferromagnetisches Material umfasst, ausgeübt werden kann. Denkbar ist auch, dass der Magnet, beispielsweise ein Elektromagnet, oberhalb der Arbeitsfläche angeordnet ist und die Schablone einen umgekehrt gepolten Magneten umfasst, so dass der Elektromagnet die Schablone bei Bestromung in Richtung der Arbeitsfläche drängt. Der umgekehrt gepolte Magnet kann auch auf die Schablone aufgelegt sein.

Die Arbeitsfläche kann auf einem, vorzugsweise umlaufenden, Transportband angeordnet sein. Vorzugsweise ist die Arbeitsfläche durch eine Oberfläche des Transportbands gebildet.

Das Abtrennen gemäß Schritt v) kann erfolgen, indem das in seiner Position auf der Arbeitsfläche gehaltene Substrat, vorzugsweise mittels des Transportbandes, entlang einer Transportrichtung gegen eine, vorzugsweise quer zur Transportrichtung erstreckte, feststehende Trenneinrichtung, vorzugsweise die als feststehende Klinge, als Bandmesser oder als rotierendes Messer ausgeführt ist, geführt wird. Mit feststehend ist dabei gemeint, dass die Trenneinrichtung ortsfest angeordnet ist.

Die Arbeitsfläche kann wenigstens eine, vorzugsweise mehrere, Erhöhung(en) und/oder Vertiefung(en) aufweisen.

Das Substrat kann ein für medizinische Zwecke geeignetes Laminat sein, wobei das Laminat mindestens eine poröse Lage, vorzugsweise die ein Textil, insbesondere ein Fasergelege und/oder ein Fasergewebe, und/oder einen Polymerschaumstoff umfasst, sowie eine Wundkontakt-Lage, die sich von der porösen Lage unterscheidet, aufweist.

Die poröse Lage kann einen Polymerschaumstoff umfassen, welcher wiederum ein Polyether, ein Polyester, ein Polyurethan, ein Polyurethan-Polyether-Copolymer, ein Polyvinylacetat, ein Polyvinylalkohol, ein Kollagen, ein Chitosan, ein Polyolefin, ein Silikon oder eine Mischung aus wenigstens zwei dieser Bestandteile umfasst.

Die Wundkontakt-Lage kann eine Vielzahl von über die Fläche verteilten Öffnungen, vorzugsweise die sich von der ersten Außenseite zur porösen Lage hin erstrecken, oder eine netzartige Struktur aufweisen.

Die Wundkontakt-Lage kann eine Salbe, ein Silikon, ein Hydrogel und/oder ein Hydrokolloid umfassen.

Die Wundkontakt-Lage kann ein Hydrogel umfassen, welches wiederum Polyacrylat, Polyurethan und/oder ein Polyurethan-Polyharnstoff-Copolymer umfasst.

Bevorzugt ist, wenn das Substrat ein Laminat ist und vorzugsweise mindestens eine die poröse Lage kontaktierende Hydrogel-Schicht umfasst. Diese Hydrogel-Schicht kann die Wundkontakt-Lage sein. Die Wundkontakt-Lage kann eine Hydrogel-Schicht umfassen oder aus einer Hydrogel-Schicht bestehen.

Hydrogel, das im als Laminat ausgebildeten Substrat gemäß dem erfindungsgemäßen Verfahren verwendet wird (beispielsweise für die Wundkontakt-Lage), umfasst bzw. ist vorzugsweise eine wasserhaltige Hydrogelmatrix. Die Hydrogelmatrix kann vorzugsweise mindestens 10 Gew.-%, insbesondere mindestens 30 Gew.-%, insbesondere mindestens 40 Gew.-% und ganz besonders bevorzugt mindestens 50 Gew.-% Wasser umfassen, wobei die Hydrogelmatrix weiterhin bevorzugt höchstens 90 Gew.-% und weiterhin bevorzugt höchsten 80 Gew.-% Wasser umfasst. Somit kann mittels des erfindungsgemäßen Verfahrens eine Wundauflage, vorzugsweise mit abgeflachten Rändern, bereitgestellt werden, welche neben einer porösen Lage eine Wundkontakt-Lage aufweist, die eine für natürliche Wundheilung ausreichende Menge Feuchtigkeit zur Verfügung stellt.

Als wasserhaltige Hydrogelmatrices können hierbei insbesondere Hydrogelmatrices verwendet werden, die eine zusammenhängende, diskrete Schicht bilden und unter Druck kein Wasser abgeben. Insbesondere sind im Zusammenhang mit der vorliegenden Erfindung Hydrogelmatrices geeignet, die ein Polyurethan-Polyharnstoff-Copolymer umfassen. Dieses Polyurethan-Polyharnstoff-Copolymer kann dabei insbesondere aus einem Präpolymer mit aliphatischen Diisocyanat-Gruppen und einem Polyamin auf Polyethylenoxidbasis gebildet werden. Insbesondere kann das Polyurethan-Polyharnstoff-Copolymer dabei aus einem Präpolymer mit Isophorondiisocyanat-Enden, einem Polyamin auf Polyethylenoxidbasis und Wasser gebildet werden. Diese Hydrogelmatrices sind besonders gut geeignet Wasser einzulagern und dieses Wasser an eine Wunde abzugeben.

Weiterhin bevorzugt kann die wasserhaltige Hydrogelmatrix weiterhin mindestens einen mehrwertigen Alkohol aus der Gruppe der zweiwertigen, dreiwertigen, vierwertigen, fünfwertigen oder sechswertigen Alkohole umfassen. Insbesondere kann der Alkohol gewählt sein aus der Gruppe der Glykole, insbesondere Ethylenglykol oder Propylenglykol, sowie Sorbitol oder Glycerin oder Mischungen hiervon. Diese Alkohole sind als Feuchtigkeitsspender geeignet und stellen somit für die die Wunde umgebende Haut eine pflegende Komponente dar.

Dabei kann die wasserhaltige Hydrogelmatrix insbesondere 0 bis 50 Gew.-% eines mehrwertigen Alkohols umfassen. Insbesondere umfasst die Hydrogelmatrix 5 bis 40 Gew.-% eines mehrwertigen Alkohols und ganz besonders bevorzugt 10 bis 30 Gew.-% eines mehrwertigen Alkohols.

Darüber hinaus kann vorgesehen sein, dass die wasserhaltige Hydrogelmatrix insbesondere mindestens ein Salz umfasst. Insbesondere ist hierbei vorgesehen, dass die Hydrogelmatrix ein anorganisches Salz umfasst. Besonders geeignet sind in diesem Zusammenhang Chloride, Iodide, Sulfate, Hydrogensulfate, Karbonate, Hydrogenkarbonate, Phosphate, Dihydrogenphosphate oder Hydrogenphosphate der Alkali- und Erdalkalimetalle. Die Hydrogelmatrix kann Natriumchlorid, Kaliumchlorid, Magnesiumchlorid, Kalziumchlorid oder Mischungen hiervon umfassen. Diese Salze simulieren das Elektrolyt-Gemisch in einem von einer Wunde abgegebenen Wundserum. Damit stellt eine diese Salze umfassende Hydrogelmatrix einer Wunde ein wundheilungsförderndes Klima zur Verfügung.

Es kann vorgesehen sein, dass die Hydrogelmatrix 0 bis 5 Gew.-% eines oder mehrerer Salze(s) umfasst. Insbesondere umfasst die Hydrogelmatrix 0,1 bis 3 Gew.-% eines Salzes und ganz besonders bevorzugt 0,5 bis 1,5 Gew.-% eines Salzes.

Im Sinne der Erfindung ist, wenn das als Laminat ausgebildete Substrat eine Hydrogelmatrix umfasst, deren Schichtdicke 0,1 bis 5,0 mm aufweist. Insbesondere weist damit eine erfindungsgemäße Wundauflage eine Wundkontakt-Lage mit einer Schichtdicke von 0,1 bis 5,0 mm, insbesondere von 0,5 bis 5,0 mm, vorzugsweise von 0,5 bis 3,0 mm auf. Wundauflagen mit Wundkontakt-Lagen mit solchen Schichtdicken zeigen einerseits keine Wundverklebung und andererseits die Fähigkeit ein von einer Wunde abgegebenes Wundexsudat aufzunehmen und an die absorbierende Schicht weiterzuleiten. Diese Schichtdicken können an jeder Stelle der Wundkontaktschicht gleich sein oder in verschiedenen Bereichen der Wundkontaktschicht verschiedene Werte annehmen.

Die Hydrogelmatrix kann Kanäle umfassen, insbesondere konische Kanäle. Die Kanäle können zum Durchtritt von Flüssigkeiten von der ersten Außenseite des Laminats, insbesondere der Wundkontakt-Lage, zur porösen Lage ausgebildet und angeordnet sein. Die Kanäle können insbesondere einen verbesserten Durchtritt für Wundexsudat bereitstellen.

Die Kanäle können einen elliptischen oder einen kreisförmigen Querschnitt aufweisen, d.h. dass die Kanäle eine kreisförmige oder elliptische Öffnung sowohl an der der ersten als auch an der zweiten Seite der Hydrogelmatrix aufweisen, wobei die kreisförmige oder elliptische Öffnung auf der ersten und der zweiten Seite verschieden groß sein können. Es kann jedoch auch vorgesehen sein, dass die Kanäle einen dreieckigen, rechteckigen, quadratischen, fünfeckigen, sechseckigen oder einen anderen vieleckigen Querschnitt aufweisen. Die erste Seite kann Öffnungen aufweisen, die im Vergleich zu der auf der zweiten Seite befindliche Öffnung größer ist.

Nach dem Zuschneiden der Ränder des Substrats und/oder dem Aufteilen des Substrats gemäß Schritt vi) kann auf die zweite Außenseite eine Backing-Lage aufgebracht werden, vorzugsweise wobei die Backing-Lage eine klebende Beschichtung aufweist und die Backing-Lage das Substrat wenigstens bereichsweise, vorzugsweise über seine gesamte flächige Erstreckung, derart überragt, dass die Substrat einen Kleberand aufweist.

Bevorzugterweise ist die erste Außenseite des Substrats durch einen ablösbaren Liner gebildet oder ein ablösbarer Liner wird, vorzugsweise nach dem Zuschneiden der Ränder des Substrats und/oder dem Aufteilen des Substrats gemäß Schritt vi) oder vor dem Auflegen des Substrats auf die Arbeitsfläche gemäß Schritt ii), auf die ersten Außenseite, vorzugsweise auf die Wundkontakt-Lage, aufgebracht.

Während mehrere Ausführungsbeispiele der vorliegenden Anmeldung die Anwendung des erfindungsgemäßen Verfahrens zur Herstellung bzw. Bearbeitung von Wundauflagen beschreiben, ist die vorliegende Erfindung hierauf nicht beschränkt.

Die vorliegende Erfindung betrifft vielmehr allgemein die Bearbeitung eines Substrats, das eine poröse-Lage umfasst. Die poröse Lage kann beispielsweise ein, insbesondere offenzelliger, Schaumstoff, vorzugsweise ein Polymerschaumstoff sein. Insbesondere bei einem Laminat zur Wundauflagen Herstellung kann die poröse Lage dabei wie nachfolgend beschrieben ausgebildet sein.

Der Polymerschaumstoff kann ein retikulierter Polymerschaumstoff sein. Der Polymerschaumstoff kann ein Polyether, ein Polyester, ein Polyurethan, ein Polyurethan-Polyether-Copolymer, ein Polyvinylacetat, ein Polyvinylalkohol, ein Kollagen, ein Chitosan, ein Polyolefin, ein Silikon oder eine Mischung aus wenigstens zwei dieser Bestandteile umfassen.

In einer Ausführungsform umfasst der Polymerschaumstoff einen offenzelligen Polyurethanschaumstoff, der erhältlich ist durch Umsetzung einer Mischung umfassend die Komponenten (i) Polyisocyanat, (ii) Polyol, bevorzugt Polyesterpolyol, (iii) Treibmittel und (iv) Katalysator.

Als Polymerschaumstoffe werden Werkstoffe mit über die gesamte Masse verteilten Zellen (offen, geschlossen oder beides) verstanden. Somit weisen solche Werkstoffe eine Rohdichte (gemäß DIN EN ISO 845) auf, die niedriger ist als die Dichte der Gerüstsubstanz.

Eine Zelle ist der bei der Herstellung von Polymerschaumstoffen gebildete einzelne Hohlraum, der von Zellwänden und/oder Zellstegen teilweise oder vollständig umschlossen ist.

Eine geschlossene Zelle ist üblicherweise eine Zelle, die vollständig von ihren Wänden umschlossen ist und daher nicht mit anderen Zellen über die Gasphase in Verbindung steht. Eine offene Zelle ist üblicherweise eine Zelle, die über die Gasphase mit anderen Zellen in Verbindung steht.

Im Rahmen dieser Anmeldung bedeutet der Begriff offenzellig, dass im Polymerschaumstoff mindestens 60 % offene Zellen, bevorzugt mindestens 90 % offene Zellen, bevorzugt mindestens 98 % offene Zellen, insbesondere im Wesentlichen 100 % offene Zellen, bezogen auf die Gesamtzahl an Zellen, vorhanden sind.

Die Offenzelligkeit des Polymerschaumstoffs wird üblicherweise nach ASTM D 2856-87, Verfahren B) bestimmt. Unter Zellwand oder Zellmembran wird üblicherweise die die Zelle umschließende Wand verstanden. Als Zellsteg wird üblicherweise der Bereich der Zellwand verstanden, der mehr als zwei Zellen voneinander trennt. Bei dem offenzelligen Polymerschaumstoff kann es sich um einen retikulierten oder um einen nicht-retikulierten Polymerschaumstoff handeln. Unter einem retikulierten Polymerschaumstoff wird ein Polymerschaumstoff verstanden, der im Wesentlichen nur Zellstege aufweist. Bei einem retikulierten Polymerschaumstoff sind die Zellwände somit im Wesentlichen entfernt.

Der Polymerschaumstoff im Sinne der vorliegenden Erfindung kann beispielsweise eine Luftdurchlässigkeit von 1000 bis 8000 l/(m²sec), mehr bevorzugt von 1500 bis 6000 l/(m²sec), noch mehr bevorzugt von 2000 bis 5000 l/(m²sec), besonders bevorzugt von 2300 bis 4000 l/(m²sec) und insbesondere von 2400 bis 3300 l/(m²sec), gemessen gemäß DIN EN ISO 9237 (20 mm Prüfdicke, 20 cm² Prüffläche, 200 Pa Differenzdruck) aufweisen.

Die Rohdichte des Polymerschaumstoffs kann im Sinne der vorliegenden Erfindung beispielsweise zwischen 10 und 350 kg/m3, gemessen gemäß DIN EN ISO 845, betragen. Bevorzugt beträgt die Rohdichte 15 bis 65 kg/m3, insbesondere 20 bis 45 kg/m3.

Es ist auch denkbar, dass die poröse Lage zwei oder mehr Lagen aus einem oder mehreren der vorgenannten Polymerschaumstoffe umfasst. Die Lagen können übereinander und/oder nebeneinander, bspw. konzentrisch zueinander, angeordnet sein.

Der in der porösen Lage vorhandene Polymerschaumstoff kann mit einem Zusatz und/oder Hilfsstoff beschichtet oder imprägniert sein. Bei dem Zusatz oder der Imprägnierung kann es sich beispielsweise um eine die Wundheilung fördernde Salbengrundlage handeln, wobei die Salbengrundlage vorzugsweise zusätzlich einen Quellstoff umfasst. Exemplarisch wird auf die WO2012/167943 verwiesen, welche im Sinne der Erfindung geeignete Salbengrundlagen beschreibt und hiermit in die Offenbarung der vorliegenden Anmeldung bezüglich der Salbengrundlagen eingebunden wird.

Alternativ oder zusätzlich kann im Polymerschaumstoff eine antimikrobiell wirksame Substanz vorhanden sein, beispielsweise Polyhexanid. Bei der antimikrobiell wirksamen Substanz kann es sich auch um ein Antibiotikum handeln, beispielsweise um Oxytetracyclin oder um eine Kombination von Bacitracin und Neomycin.

Gegebenenfalls kann der Zusatz oder die Imprägnierung zusätzlich zum Antibiotikum oder alternativ eine entzündungshemmende Substanz umfassen, beispielsweise Hydrocortison. Gemäß einer Ausführungsform umfasst die antimikrobiell wirksame Substanz Silber oder eine Silber-Verbindung.

Die poröse Lage, insbesondere der Polymerschaumstoff, kann Silber, vorzugsweise in Form von Silberionen oder in Form von atomarem Silber, enthalten. Bevorzugt ist Silber in Form einer Silberbeschichtung auf der Oberfläche der porösen Lage aufgebracht. Alternativ kann das Silber innerhalb der porösen Lage verteilt sein.

Beispielsweise kann bei offenzelligen Polymerschaumstoffen Silber bereits in die härtbare Zusammensetzung des Polymers mit eingebracht werden. Bevorzugt enthält das im Polymerschaumstoff vorhandene Polymer 0,000001 bis 0,1 Gew. % Silber, mehr bevorzugt 0,0001 bis 0,01 Gew.-% Silber, bezogen auf das Gesamtgewicht des jeweiligen Polymerschaumstoffs. Besonders bevorzugt enthält der Polymerschaumstoff 0,0001 bis 0,01 Gew.-% Silber, bezogen auf das Gesamtgewicht des Polymerschaumstoffs.

Des Weiteren kann die poröse Lage einen, insbesondere Partikel-förmigen, Zusatz umfassen, wobei es sich bei dem, insbesondere Partikel-förmigen, Zusatz insbesondere um ein quellfähiges Polymer handeln kann. Gemäß einer Ausführungsform umfasst die poröse Lage ein superabsorbierendes Polymer (SAP), insbesondere Partikel aus einem superabsorbierenden Polymer.

Die eben beschriebenen Ausgestaltungen der porösen Lage eignen sich besonders für die Herstellung von Wundauflagen, die im Rahmen des erfindungsgemäßen Verfahrens bearbeitet werden können.

Weitere Merkmale, Anwendungsmöglichkeiten und Vorteile der Erfindung ergeben sich aus der nachfolgenden Beschreibung von Ausführungsbeispielen der Erfindung, die anhand der Zeichnung erläutert werden, wobei die Merkmale sowohl in Alleinstellung als auch in unterschiedlicher Kombination für die Erfindung wichtig sein können, ohne dass hierauf nochmals explizit hingewiesen wird. Es zeigen:
- Figur 1: einen Ausschnitt aus einem Substrat, das aus einem Laminat, das für medizinische Zwecke geeignet ist, gebildet ist;
- Figur 2: eine schematische Darstellung der Durchführung von Schritten des Verfahrens;
- Figur 3: eine schematische Illustration einer weiteren Ausführungsform des erfindungsgemäßen Verfahrens;
- Figur 4: eine schematische Illustration weiteren einer Ausführungsform des erfindungsgemäßen Verfahrens;
- Figur 5: eine schematische Illustration weiteren einer Ausführungsform des erfindungsgemäßen Verfahrens; und
- Figur 6: eine Illustration eines Verfahrensschritts vi).

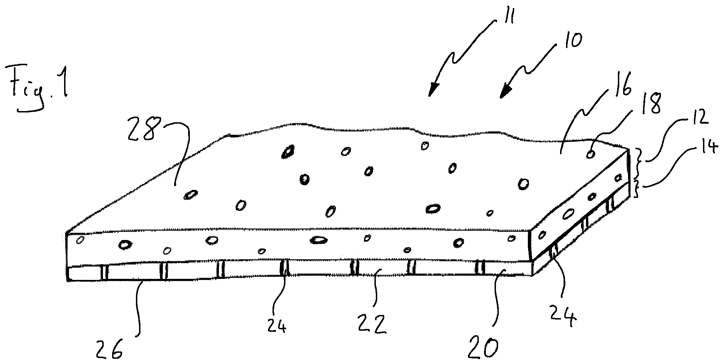

Figur 1 zeigt ein Laminat 10, das für medizinische Zwecke geeignet ist, und ein Substrat 11 wie es typischerweise zur Durchführung des erfindungsgemäßen Verfahrens Verwendung finden kann darstellt. Das Laminat 10 ist vorliegend nur bereichsweise dargestellt.

Das Substrat 11 bzw. Laminat 10 umfasst in der in Figur 1 gezeigten Ausführungsform eine poröse Lage 12 und eine Wundkontaktlage 14. Die poröse Lage 12 ist vorliegend durch einen offenzelligen Polymerschaumstoff 16 gebildet. An den Oberflächen des Polymerschaumstoffs 16 sind Poren 18 symbolisch angedeutet.

Die Wundkontaktlage 14 ist vorliegend als Hydrogelschicht 20 ausgebildet. Andere Arten von Wundkontaktlagen sind ebenso denkbar. Die Hydrogelschicht 20 ist vorliegend als wasserhaltige Hydrogelmatrix 22 ausgebildet. Die als Hydrogelschicht 20 ausgebildete Wundkontaktlage 14 steht in direktem Kontakt mit der porösen Lage 12.

Die Wundkontaktlage 14 weist eine Vielzahl von über die Fläche der Wundkontaktlage 14 verteilten Öffnungen 24 auf, die in Figur 1 symbolisch dargestellt sind. Anstatt oder zusätzlich zu den Öffnungen 24 kann die Wundkontaktlage 14 auch eine netzartige Struktur aufweisen.

Die Öffnungen 24 und/oder die netzartige Struktur sind vorteilhafterweise derart ausgebildet, dass Flüssigkeit von einer ersten Außenseite 26 des Laminats 10 durch die Wundkontaktlage 14 zur porösen Lage 12 über Kapillarkräfte transportiert werden kann.

Eine zweite Außenseite 28 des Substrat 11 bzw. des Laminats 10 wird im vorliegenden Beispiel durch die der Wundkontaktlage 14 abgewandten Seite der porösen Lage 12 gebildet.

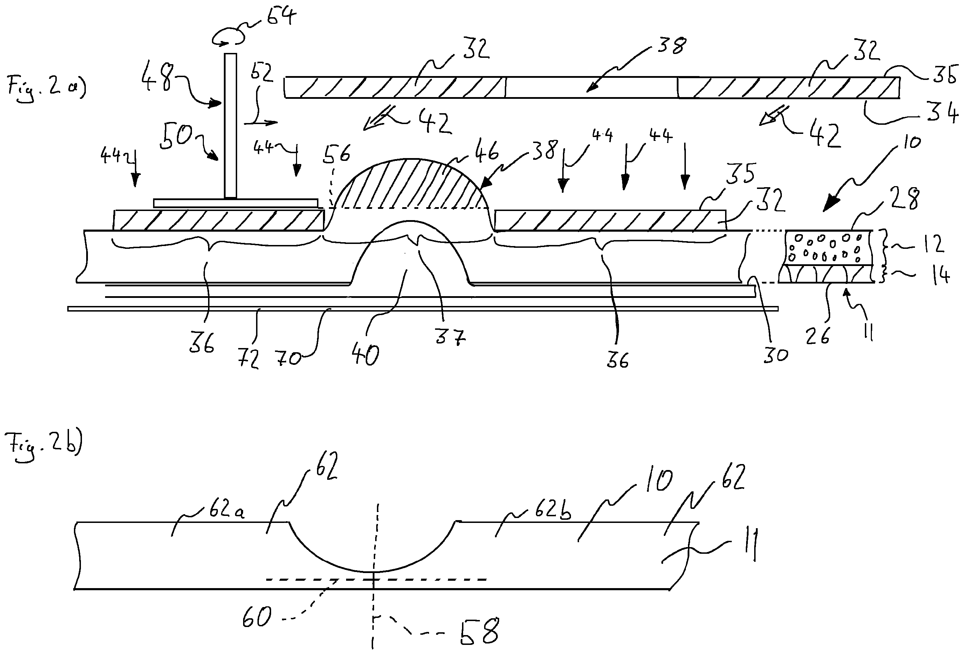

In Figur 2 ist die Durchführung des erfindungsgemäßen Verfahrens schematisch illustriert. In einem ersten Schritt i) wurde das Substrat 11 bereitgestellt.

In einem zweiten Schritt wurde das Substrat 11 bzw. das Laminat 10 mit seiner ersten Außenseite 26 auf eine Arbeitsfläche 30 aufgelegt. Die erste Außenseite 26 kontaktiert in diesem Zustand die Arbeitsfläche 30 flächig.

In einem dritten Schritt iii) wurde eine Schablone 32, die eine Kontaktseite 34 sowie eine Oberseite 35 umfasst, aufgebracht. Die Schablone 32 deckt einen bedeckten Teilbereich 36 des Substrats 11 ab und ein freier Teilbereich 37 des Substrats 11 bleibt von der Schablone 32 unbedeckt.

Die Schablone 32 weist einen Durchbruch 38 auf. Die Arbeitsfläche 30 weist eine Erhöhung 40 auf. Beim Platzieren der Schablone 32, welches in Figur 2a) über Pfeile mit dem Bezugszeichen 42 symbolisch dargestellt ist, wird der Durchbruch 38 über der Erhöhung 40 platziert. Im vorliegenden Beispiel drängt die Erhöhung 40 das Substrat 11 durch den Durchbruch 38 der Schablone 32 hindurch, so dass ein Teil des Substrats 11 bzw. ein Teil 46 des freien Teilbereichs 37 über die Oberseite 35 der Schablone 32 hinausragt.

In einem vierten Schritt iv) des Verfahrens wird die Schablone 32 in Richtung der Arbeitsfläche 30 angepresst, was durch Pfeile mit dem Bezugszeichen 44 symbolisch dargestellt ist.

Ein Magnet 70 ist unterhalb der Arbeitsfläche 30 angeordnet. Der Magnet 70 ist vorliegend als Elektromagnet 72 ausgebildet.

Ist der Elektromagnet 72 bestromt, so zieht er die ein ferromagnetisches Material umfassende Schablone 32 magnetisch an die Arbeitsfläche 30 heran. Dieses magnetkraftbasierte Heranziehen stellt das Anpressen im Schritt iv) dar.

Durch das Anpressen wird im Beispiel von Figur 2 das Substrat 11 in seiner Position auf der Arbeitsfläche 30 gehalten. Im Beispiel von Figur 2 wird das Substrat 11 dabei im Wesentlichen nicht komprimiert.

In einem fünften Schritt v) wird wenigstens ein Teil des freien Teilbereichs 37 des Substrats 11 bearbeitet. Vorliegend wird der Teil 46 des freien Teilbereichs 37, der über die Oberseite 35 der Schablone 32 hinausragt abgetrennt. Der abgetrennte bzw. abzutrennende Teil 46 ist in Figur 2a schraffiert dargestellt.

Denkbar ist auch, dass das Bearbeiten im fünften Schritt v) das Aufbringen einer Substanz auf den freien Teilbereich 37 des Substrats 11 umfasst. Alternativ oder zusätzlich ist auch denkbar, dass das Bearbeiten im fünften Schritt v) umfasst, den freien Teilbereich 37 des Substrats 11 einer Strahlung, beispielsweise Licht, insbesondere UV-Licht oder Infrarotlicht, auszusetzen.

Das im Beispiel von Figur 2 gezeigte Abtrennen gemäß dem fünften Schritt v) erfolgt im Beispiel von Figur 2 mittels einer Trenneinrichtung 48. In dem Beispiel von Figur 2a ist die Trenneinrichtung 48 als verfahrbares rotierendes Messer 50 ausgebildet. Die Verfahrbarkeit des Messers 50 ist durch einen Pfeil mit dem Bezugszeichen 52 dargestellt und die Rotation des Messers 50 durch einen Pfeil mit dem Bezugszeichen 54.

Das Abtrennen des Teils 46 des Laminats 10 bzw. des Substrats 11 erfolgt in einer Trennebene 56, die in Figur 2a durch eine unterbrochene Linie symbolisch dargestellt ist. Die Trennebene 56 ist vorliegend im Wesentlichen parallel zur Erstreckung der Arbeitsfläche 30 sowie zur Erstreckung der Schablone 32 angeordnet. Die Trennebene 56 ist im vorliegenden Beispiel weniger als 2 mm zur Oberseite 35 der Schablone 32 beabstandet. Im Sinne der Erfindung ist jedoch auch eine größere Beabstandung der Trennebene 56 zur Oberseite 35 der Schablone 32 möglich.

In einem sechsten Schritt vi) wird das Substrat 11 bzw. das Laminat 10 entlang einer Aufteillinie 58 aufgeteilt, um mehrere Teilstücke, vorliegend die Teilsubstrate 62a und 62b zu erhalten. Die Aufteillinie 58 erstreckt sich im Wesentlichen vertikal zur Erstreckungsebene 60 des Laminats 10. Durch die Aufteilung entlang der Aufteillinie 58 wird das Substrat 11 bzw. Laminat 10 vorliegend in mehrere Wundauflagen 62 aufgeteilt, die durch die Teilsubstrate 62a und 62b gebildet sind, siehe Figur 2b.

Bspw. im Anschluss an das Zuschneiden bzw. bereits im Anschluss an den Schritt v) kann das Aufbringen einer flächigen Materialschicht auf die erste Außenseite 26 und/oder die zweite Außenseite 28 des Substrats 11 oder eines der Teilsubstrate 62a und 62b erfolgen.

Bspw. kann das Substrat 11 bzw. das Laminat 10 bzw. die Wundauflagen 62 mit einer wundheilungsfördernden Substanz oder Zusammensetzung in Form einer flächigen Materialschicht beaufschlagt werden und/oder das Laminat 10 bzw. das Substrat 11 kann mit einer wundheilungsfördernden Substanz oder Zusammensetzung imprägniert werden.

In einem Schritt ix) kann das Substrat 11 bzw. das Laminat 10 bzw. die Wundauflagen 62 sterilisiert und verpackt werden.

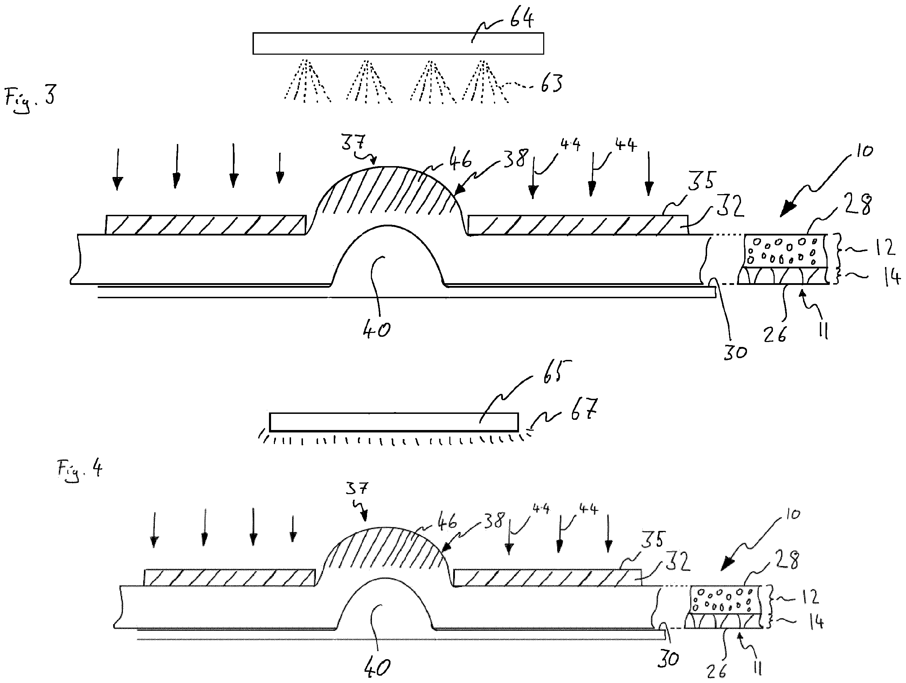

In Figur 3 ist eine Alternative des Verfahrens gezeigt. In der Alternative von Figur 3 wird das Substrat 11 bzw. dessen freier Teilbereich 37 im Schritt v) bearbeitet, in dem das Bearbeiten des freien Teilbereichs 37 des Substrats 11 das Aufbringen einer Substanz 63 auf den freien Teilbereich 37 des Substrats 11 umfasst. Vorliegend wird die Substanz 63 auf das Substrat 11 bzw. auf den freien Teilbereich 37 des Substrats 11 mittels einer Sprühanlage 64 aufgesprüht.

In Figur 4 ist eine weitere Alternative des Verfahrens gezeigt. In der Alternative von Figur 4 wird das Substrat 11 bzw. dessen freier Teilbereich 37 im Schritt v) bearbeitet, indem der freie Teilbereich 37 des Substrats 11 einer Strahlung 67 aus einer Strahlungsquelle 65 ausgesetzt wird.

Es ist auch möglich, dass auf den freien Teilbereich 37 zunächst eine Substanz 63 aufgebracht wird, wie in Figur 3 gezeigt und nachfolgend der freie Teilbereich 37 des Substrats 11 einer Strahlung 67 aus einer Strahlungsquelle 65 ausgesetzt wird, wie in Figur 4 dargestellt. So ist es bspw. denkbar, eine Substanz 63 aufzubringen, die durch eine nachfolgende Strahlung 67 aus einer Strahlungsquelle 65 modifiziert, insbesondere gehärtet, wird. Bei der Strahlung 67 kann es sich bspw. um sichtbares Licht, UV-Licht oder Infrarotlicht handeln.

Jedenfalls die Schritte i) bis iv) des Verfahrens aus den Figuren 3 und 4 sind im vorliegenden Beispiel mit denen des in Figur 2 illustrierten Verfahrens identisch und werden nicht nochmals erläutert.

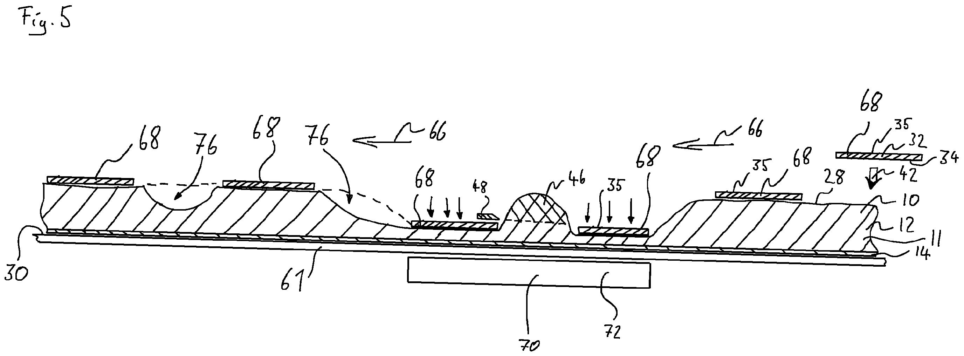

In Figur 5 ist eine weitere Alternative des Verfahrens gezeigt. Es ist in Figur 5 gezeigt, wie zunächst die Schablone 32 auf das Substrat 11 aufgebracht wird, was durch den Pfeil mit dem Bezugszeichen 42 schematisch angedeutet ist.

Im Ausführungsbeispiel von Figur 5 ist die Arbeitsfläche 30 als Oberseite eines Transportbands 61 ausgeführt. Das Transportband 61 bewegt das Substrat 11 entlang einer Transportrichtung 66. Die Transportrichtung 66 ist in Figur 5 durch Pfeile mit entsprechendem Bezugszeichen symbolisch dargestellt. Die Schablone 32 ist im Ausführungsbeispiel von Figur 5 durch mehrere miteinander unverbundene Schablonenabschnitte 68 gebildet.

Die einzelnen Schablonenabschnitte 68 werden in einer gewünschten Anordnung zueinander auf das Substrat 11 aufgebracht, so dass sie jeweils die zweite Außenseite 28 des Substrats 11 mit ihrer Kontaktseite 34 kontaktieren. Nachdem die Schablonen 32 bzw. die einzelnen Schablonenabschnitte 68 auf das Substrat 11 aufgebracht sind, bewegt das Transportband 61 das Substrat 11 mit den darauf liegenden Schablonenabschnitten 68 entlang der Transportrichtung 66.

In Transportrichtung 66 auf den Aufbringort der Schablonenabschnitte 68 nachfolgend ist ein Magnet 70 unterhalb der Arbeitsfläche 30 angeordnet. Der Magnet 70 ist vorliegend als Elektromagnet 72 ausgebildet. Ist der Elektromagnet 72 bestromt, so zieht er die ein ferromagnetisches Material umfassenden Schablonenabschnitte 68 magnetisch an die Arbeitsfläche 30 heran. Durch dieses Heranziehen wird die poröse Lage 12 des Substrats 11 in seiner Erstreckung von der Arbeitsfläche zur zweiten Außenseite 28 hin komprimiert.

In den Zwischenräumen zwischen den einzelnen Schablonenabschnitten 68, die Durchbrüche 38 bilden, wird das Substrat 11 weniger komprimiert als unterhalb der Schablonenabschnitte 68. Die über die Oberseite 36 der Schablonenabschnitte 68 herausragenden Teile 46 des Laminats 10 werden durch die Bewegung des Transportbandes 61 gegen eine Trenneinrichtung 48, die vorliegend als feststehende Klinge ausgeführt ist, geführt. Hierdurch werden die über die Oberseite 36 der Schablonenabschnitte 68 überstehenden Teile des Substrats 11 abgetrennt. Wenn die Schablonenabschnitte 68 durch die Bewegung des Transportbandes 61 entlang der Transportrichtung 66 von dem Magneten 70 wegbewegt werden, so dehnt sich das Substrat 11 wieder in seine ursprüngliche Erstreckung aus und es ist ersichtlich, dass an den Stellen, an denen die Teile 46 des Substrat 11 abgetrennt wurden, das Laminat nun eine Ausnehmung bzw. Mulde 76 aufweist.

Je nach Ausbildung und Anordnung der Durchbrüche 38 in der Schablone 32 oder einzelner Schablonenabschnitte 68 der Schablone 32 können komplexe dreidimensionale Strukturen in das Substrat 11 eingebracht werden.

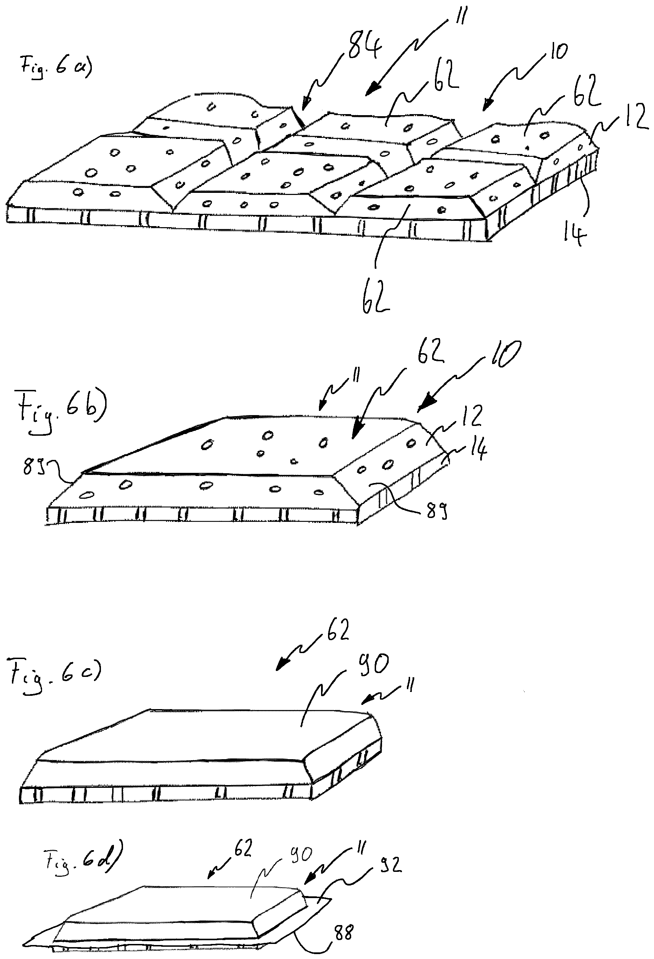

In Figur 6a ist eine beispielhafte Konturierung eines Substrats 11 gezeigt. Das Substrat 11 weist quer und längs verlaufende Vertiefungen 84 auf. Die Vertiefungen 84 erstrecken sich durch die poröse Lage 12 des als Laminat 10 ausgeführten Substrats 11 und sind im Rahmen der Bearbeitung in einem Schritt v) in die poröse Lage 12 des als Laminat 10 ausgebildeten Substrats 11 eingebracht worden.

In einem Schritt vi) kann das Substrat 11, im Beispiel von Figur 6a und 6b entlang der Vertiefungen 84, vollständig durch einen im Wesentlichen vertikal zur Erstreckungsebene 60 des Substrats 11 verlaufenden Aufteilvorgang gemäß einem Schritt vi) in einzelne Teilsubstrate 62, die bspw. Wundauflagen sein können, aufgeteilt werden.

Das Teilsubstrat 62 weist, wie in Figur 6c gezeigt, abgeschrägte Kanten 89 bzw. Seitenflächen 89 auf. In Figur 6c ist auch gezeigt, dass auf das Teilsubstrat 62 aus Figur 6b eine Backing-Lage 90 aufgebracht werden kann. Die Backing-Lage 90 weist eine klebende Beschichtung 88 auf. Die Backing-Lage 90 ist im Beispiel von Figur 6c als Polyurethanfilm ausgebildet und auf der zweiten Außenseite 28 des Teilsubstrats 62 aufgebracht.

Anders als im Beispiel von Figur 6c gezeigt, kann die Backing-Lage 90 das Teilsubstrat 62 auch wenigstens bereichsweise vorzugsweise über seine gesamte flächige Erstreckung derart überragen, dass das Teilsubstrat 62, welches vorliegend eine Wundauflage bildet einen Kleberand 92 aufweist. Ein Teilsubstrat 62 bzw. eine entsprechende Wundauflage mit Backing-Lage 90, die einen Kleberand 92 bildet, ist in Figur 6d illustriert.

## Patentansprüche

1. Verfahren zur Bearbeitung eines, vorzugsweise für medizinische Zwecke geeigneten, flächigen Substrats (11), das eine poröse-Lage (12) umfasst, insbesondere die wiederum eine Schaumstoff-Lage oder ein Textil, insbesondere ein Fasergelege und/oder ein Fasergewebe, umfasst, wobei das Verfahren die Schritte umfasst:
Schritt i) Bereitstellen des Substrats (11), wobei das Substrat (11) eine erste Außenseite (26) und eine zweite Außenseite (28) aufweist,
Schritt ii) Auflegen des Substrats (11) auf eine Arbeitsfläche (30), wobei die erste Außenseite (26) die Arbeitsfläche (30) nach dem Auflegen flächig kontaktiert,
Schritt iii) Aufbringen einer Schablone (32), die eine Kontaktseite (34) und eine Oberseite (35) aufweist, vorzugsweise wobei die Schablone (32) mindestens einen Durchbruch (38) aufweist, wobei die Kontaktseite (34) der Schablone (32) beim Aufbringen auf die der Arbeitsfläche (30) abgewandte zweite Außenseite (28) des Substrats (11) aufgebracht wird, wobei die Schablone (32) einen bedeckten Teilbereich (36) des Substrats (11) abdeckt und ein freier Teilbereich (37) des Substrats (11) von der Schablone (32) unbedeckt ist,
Schritt iv) Anpressen der Schablone (32) in Richtung der Arbeitsfläche (30) mittels einer direkt auf die Schablone (32) wirkenden magnetischen Kraft, wobei die Schablone (32) zu diesem Zweck ferromagnetisches Material umfasst, wobei das Substrat (11) durch das Anpressen der Schablone (32) in seiner Position auf der Arbeitsfläche (30) gehalten, vorzugsweise und in seiner Erstreckung von der ersten Außenseite (26) zur zweiten Außenseite (28) komprimiert, wird, wobei in oder unter der Arbeitsfläche (30) ein Permanentmagnet und/oder ein Elektromagnet (72) angeordnet ist bzw. sind, so dass mittels des Permanentmagneten und/oder des Elektromagneten (72) die zur Arbeitsfläche (30) hin orientierte magnetische Kraft auf die Schablone (32) ausgeübt werden kann,
Schritt v) Bearbeiten wenigstens eines Teils (46) des freien Teilbereichs (37) des Substrats (11), vorzugsweise wobei das Bearbeiten ein Abtrennen eines Teils (46) des freien Teilbereichs (37) des Substrats (11), der über die Oberseite (35) der Schablone (32) hinausragt umfasst, und/oder wobei das Bearbeiten ein Aufbringen einer Substanz (63) auf den freien Teilbereich (37) des Substrats (11) umfasst,
vorzugsweise Schritt vi) Zuschneiden der Ränder des Substrats (11) und/oder Aufteilen des Substrats (11), um mehrere Teilstücke zu erhalten, wobei das Zuschneiden und/oder Aufteilen insbesondere durch einen im Wesentlichen vertikal zu der Erstreckungsebene (60) des Substrats (11) geführten Schnitt erfolgt,
vorzugsweise Schritt vii) Aufbringen einer flächigen Materialschicht auf die erste Außenseite (26) und/oder die zweite Außenseite (28) des Substrats (11) oder eines der Teilstücke,
vorzugsweise Schritt viii) Sterilisieren und/oder Verpacken des Substrats (11) oder eines Teilstücks des Substrats (11),
vorzugsweise wobei zwei, vorzugsweise mehrere, vorzugsweise sämtliche Schritte i) bis viii) in der eben genannten Reihenfolge erfolgen.

2. Verfahren nach Anspruch 1, wobei das Verfahren weiterhin wenigstens umfasst
Beaufschlagen und/oder Imprägnieren des Substrats (11) mit einer, vorzugsweise wundheilungsfördernden, Substanz (63)oder Zusammensetzung.

3. Verfahren nach Anspruch 1 oder 2, wobei die Schablone (32) einstückig, vorzugsweise mit einem Durchbruch (38), ausgebildet ist oder mehrstückig ausgebildet ist und mehrere Schablonenteile umfasst, vorzugsweise wobei die Schablonenteile derart auf das Substrat (11) aufgelegt sind, dass zwischen den Schablonenteilen ein Durchbruch (38) gebildet ist.

4. Verfahren nach einem oder mehreren der voranstehenden Ansprüche, wobei die Arbeitsfläche (30) auf einem, vorzugsweise umlaufenden, Transportband (61) angeordnet ist, vorzugsweise durch eine Oberfläche des Transportbands (61) gebildet ist.

5. Verfahren nach einem oder mehreren der voranstehenden Ansprüche, wobei das Abtrennen gemäß Schritt v) erfolgt, indem das in seiner Position auf der Arbeitsfläche (30) gehaltene Substrat (11), vorzugsweise mittels des Transportbandes (61), entlang einer Transportrichtung (66) gegen eine, vorzugsweise quer zur Transportrichtung (66) erstreckte, feststehende Trenneinrichtung (48), vorzugsweise die als feststehende Klinge, als Bandmesser oder als rotierendes Messer ausgeführt ist, geführt wird.

6. Verfahren nach einem oder mehreren der voranstehenden Ansprüche, wobei die Arbeitsfläche (30) wenigstens eine, vorzugsweise mehrere, Erhöhung(en) (40) und/oder Vertiefung(en) aufweist.

7. Verfahren nach einem oder mehreren der voranstehenden Ansprüche, wobei das Substrat (11) ein für medizinische Zwecke geeignetes Laminat (10) ist, wobei das Laminat (10) mindestens eine poröse-Lage (12), vorzugsweise die ein Textil, insbesondere ein Fasergelege und/oder ein Fasergewebe, und/oder einen Polymerschaumstoff umfasst, sowie eine Wundkontakt-Lage (14), die sich von der porösen Lage (12) unterscheidet, aufweist

8. Verfahren nach dem voranstehenden Anspruch, wobei die poröse-Lage (12) einen Polymerschaumstoff umfasst, welcher wiederum ein Polyether, ein Polyester, ein Polyurethan, ein Polyurethan-Polyether-Copolymer, ein Polyvinylacetat, ein Polyvinylalkohol, ein Kollagen, ein Chitosan, ein Polyolefin, ein Silikon oder eine Mischung aus wenigstens zwei dieser Bestandteile umfasst.

9. Verfahren nach einem oder mehreren der beiden voranstehenden Ansprüche, wobei die Wundkontakt-Lage (14) eine Vielzahl von über die Fläche verteilten Öffnungen (24), vorzugsweise die sich von der ersten Außenseite (26) zur porösen Lage (12) hin erstrecken, oder eine netzartige Struktur aufweist.

10. Verfahren nach einem oder mehreren der 3 voranstehenden Ansprüche, wobei die Wundkontakt-Lage (14) eine Salbe, ein Silikon, ein Hydrogel und/oder ein Hydrokolloid umfasst.

11. Verfahren nach einem oder mehreren der 4 voranstehenden Ansprüche, wobei die Wundkontakt-Lage ein Hydrogel umfasst, welches wiederum Polyacrylat, Polyurethan und/oder ein Polyurethan-Polyharnstoff-Copolymer umfasst.

12. Verfahren nach einem oder mehreren der voranstehenden Ansprüche, wobei nach dem Zuschneiden der Ränder des Substrats (11) und/oder dem Aufteilen des Substrats (11) gemäß Schritt vi) auf die zweite Außenseite (28) eine Backing-Lage (90) aufgebracht wird, vorzugsweise wobei die Backing-Lage (90) eine klebende Beschichtung (88) aufweist und die Backing-Lage (90) das Substrat (11) wenigstens bereichsweise, vorzugsweise über seine gesamte flächige Erstreckung, derart überragt, dass die Substrat (11) einen Kleberand (92) aufweist.

13. Verfahren nach einem oder mehreren der voranstehenden Ansprüche, wobei die erste Außenseite (26) des Substrats (11) durch einen ablösbaren Liner gebildet ist oder wobei ein ablösbarer Liner, vorzugsweise nach dem Zuschneiden der Ränder des Substrats (11) und/oder dem Aufteilen des Substrats (11) gemäß Schritt vi) oder vor dem Auflegen des Substrats (11) auf die Arbeitsfläche (30) gemäß Schritt ii), auf die ersten Außenseite (26), vorzugsweise auf die Wundkontakt-Lage (14), aufgebracht wird.

## Claims

1. Method for processing a planar substrate (11), preferably suitable for medical purposes, comprising a porous layer (12), in particular which in turn comprises a foam layer or a textile, in particular a fiber stack and/or a fiber fabric, wherein the method comprises the steps of:
step i) providing the substrate (11), wherein the substrate (11) has a first outer side (26) and a second outer side (28),
step ii) placing the substrate (11) onto a work surface (30), wherein the first outer side (26) contacts the work surface (30) in a planar manner after the placement,
step iii) applying a template (32) which has a contact side (34) and an upper side (35), wherein the template (32) preferably has at least one opening (38), wherein the contact side (34) of the template (32) is applied during application to the second outer side (28) of the substrate (11) facing away from the working surface (30), wherein the template (32) covers a covered partial region (36) of the substrate (11) and a free partial region (37) of the substrate (11) is uncovered by the template (32),
step iv) pressing the template (32) in the direction of the work surface (30) by means of a magnetic force acting directly on the template (32), wherein the template (32) comprises ferromagnetic material for this purpose, wherein the substrate (11) is held in its position on the work surface (30) by pressing the template (32) and preferably compressed in its extension from the first outer side (26) to the second outer side (28), wherein a permanent magnet and/or an electromagnet (72) is/are arranged in or below the work surface (30) so that the magnetic force oriented toward the work surface (30) can be exerted on the template (32) by means of the permanent magnet and/or the electromagnet (72),
step v) processing at least a part (46) of the free partial region (37) of the substrate (11), wherein processing preferably comprises separating a part (46) of the free partial region (37) of the substrate (11) which projects beyond the upper face (35) of the template (32), and/or wherein processing comprises applying a substance (63) to the free partial region (37) of the substrate (11),
preferably step vi) cutting the edges of the substrate (11) and/or dividing the substrate (11) in order to obtain a plurality of pieces, wherein cutting and/or dividing takes place in particular by a cut guided substantially vertically to the extension plane (60) of the substrate (11),
preferably step vii) applying a planar material layer to the first outer side (26) and/or the second outer side (28) of the substrate (11) or one of the pieces,
preferably step viii) sterilising and/or packaging the substrate (11) or a piece of the substrate (11),
wherein two of, preferably a plurality of, preferably all of, steps i) to viii) preferably occur in the sequence just mentioned.

2. Method according to claim 1, wherein the method further comprises at least
applying and/or impregnating the substrate (11) with a substance (63) or composition which preferably promotes wound healing.

3. Method according to either claim 1 or claim 2, wherein the template (32) is formed integrally, preferably with an opening (38), or is multi-piece and comprises a plurality of template parts, wherein the template parts are preferably placed on the substrate (11) such that an opening (38) is formed between the template parts.

4. Method according to one or more of the preceding claims, wherein the work surface (30) is arranged on a preferably revolving conveyor belt (61), preferably formed by a surface of the conveyor belt (61).

5. Method according to one or more of the preceding claims, wherein separation according to step v) is carried out by guiding the substrate (11) held in its position on the work surface (30), preferably by means of the conveyor belt (61), along a transport direction (66) against a stationary separating device (48), preferably extending transversely to the transport direction (66), preferably which is designed as a stationary blade, as a band knife or as a rotating blade.

6. Method according to one or more of the preceding claims, wherein the work surface (30) has at least one, preferably a plurality of, elevation(s) (40) and/or depression (s) .

7. Method according to one or more of the preceding claims, wherein the substrate (11) is a laminate (10) suitable for medical purposes, wherein the laminate (10) is at least one porous layer (12), preferably which comprises a textile, a fiber stack and/or a fiber fabric, and/or a polymer foam, and a wound-contacting layer (14) which differs from the porous layer (12).

8. Method according to the preceding claim, wherein the porous layer (12) comprises a polymer foam which in turn comprises a polyether, a polyester, a polyurethane, a polyurethane-polyether copolymer, a polyvinyl acetate, a polyvinyl alcohol, a collagen, a chitosan, a polyolefin, a silicone or a mixture of at least two of these constituents.

9. Method according to one or more of the two preceding claims, wherein the wound-contacting layer (14) has a plurality of openings (24) which are distributed over the surface, preferably which extend from the first outer side (26) toward the porous layer (12), or has a net-like structure.

10. Method according to one or more of the 3 preceding claims, wherein the wound-contacting layer (14) comprises an ointment, a silicone, a hydrogel and/or a hydrocolloid.

11. Method according to one or more of the 4 preceding claims, wherein the wound-contacting layer comprises a hydrogel which in turn comprises polyacrylate, polyurethane and/or a polyurethane-polyurea copolymer.

12. Method according to one or more of the preceding claims, wherein, after cutting the edges of the substrate (11) and/or dividing the substrate (11) according to step vi), a backing layer (90) is applied to the second outer side (28), wherein the backing layer (90) preferably has an adhesive coating (88) and the backing layer (90) projects beyond the substrate (11) at least in regions, preferably over its entire planar extension, such that the substrate (11) has an adhesive edge (92).

13. Method according to one or more of the preceding claims, wherein the first outer side (26) of the substrate (11) is formed by a detachable liner or wherein a detachable liner, preferably after the edges of the substrate (11) are cut and/or the substrate (11) is divided according to step vi) or before the substrate (11) is placed on the work surface (30) according to step ii), is applied to the first outer side (26), preferably to the wound-contacting layer (14) .

## Revendications

1. Procédé de traitement d'un substrat plat (11), de préférence adapté à des fins médicales, qui comprend une couche poreuse (12), en particulier qui comprend à son tour une couche de mousse ou un textile, en particulier une nappe en fibres et/ou un tissu de fibres, dans lequel le procédé comprend les étapes:
étape i) consistant à fournir le substrat (11), dans lequel le substrat (11) présente une première face extérieure (26) et une deuxième face extérieure (28),
étape ii) consistant à placer le substrat (11) sur une surface de travail (30), dans lequel, après l'avoir placé, la première face extérieure (26) entre en contact plan avec la surface de travail (30),
étape iii) consistant à appliquer un gabarit (32) qui présente une face de contact (34) et une face supérieure (35), de préférence dans lequel ledit gabarit (32) présente au moins une ouverture (38), dans lequel la face de contact (34) du gabarit (32) est appliqué lors de l'application sur la deuxième face extérieure (28) du substrat (11), qui montre dans la direction opposée à la surface de travail (30), dans lequel le gabarit (32) couvre une zone partielle couverte (36) du substrat (11) et une zone partielle libre (37) du substrat (11) n'est couverte par le gabarit (32),
étape iv) consistant à presser le gabarit (32) en direction de la surface de travail (30) au moyen d'une force magnétique agissant directement sur le gabarit (32), dans lequel, à cette fin, le gabarit (32) comprend du matériau ferromagnétique, dans lequel le substrat (11) est maintenu dans sa position sur la surface de travail (30) par le fait de presser le gabarit (32), de préférence, et dans son extension de la première face extérieure (26) à la deuxième face extérieure (28), est comprimé, dans lequel un aimant permanent et/ou un électro-aimant (72) est ou bien sont agencé(s) dans ou sous la surface de travail (30) de sorte que la force magnétique orientée vers la surface de travail (30) peut être exercée sur le gabarit (32) au moyen de l'aimant permanent et/ou de l'électro-aimant (72),
étape v) consistant à traiter au moins une partie (46) de la zone partielle libre (37) du substrat (11), de préférence dans lequel le traitement comprend un détachement d'une partie (46) de la zone partielle libre (37) du substrat (11), qui dépasse la face supérieure (35) du gabarit (32), et/ou dans lequel le traitement comprend une application d'une substance (63) sur la zone partielle libre (37) du substrat (11),
de préférence l'étape vi) consistant à couper les bords du substrat (11) et/ou à diviser le substrat (11) pour obtenir plusieurs sections, dans lequel l'action de couper et/ou la division s'effectue en particulier par une coupe réalisée pour l'essentiel verticalement au plan d'extension (60) du substrat (11),
de préférence l'étape vii) consistant à appliquer une couche plane de matériau sur la première face extérieure (26) et/ou la deuxième face extérieure (28) du substrat (11) ou de l'une des sections,
de préférence l'étape viii) consistant à stériliser et/ou à emballer le substrat (11) ou une section du substrat (11),
de préférence dans lequel deux, de préférence plusieurs, de préférence toutes les étapes i) à viii) s'effectuent dans l'ordre qui vient d'être mentionné.

2. Procédé selon la revendication 1, dans lequel le procédé comprend en outre au moins
appliquer au et/ou imprégner le substrat (11) (d')une substance (63) ou (d')une composition qui favorise de préférence la cicatrisation des plaies.

3. Procédé selon la revendication 1 ou 2, dans lequel le gabarit (32) est formé d'une seule pièce, de préférence avec une ouverture (38), ou est réalisé en plusieurs pièces et comprend une pluralité de parties de gabarit, de préférence dans lequel les parties de gabarit sont placés sur le substrat (11) de telle manière qu'une ouverture (38) est formée entre les parties de gabarit.

4. Procédé selon l'une ou plusieurs des revendications précédentes, dans lequel la surface de travail (30) est agencée sur une bande transporteuse (61) de préférence circulante, de préférence est formée par une surface de la bande transporteuse (61).

5. Procédé selon l'une ou plusieurs des revendications précédentes, dans lequel le détachement selon l'étape v) se fait en guidant le substrat (11) maintenu dans sa position sur la surface de travail (30), de préférence au moyen de la bande transporteuse (61), le long d'une direction de transport (66) contre un dispositif de séparation fixe (48) qui s'étend de préférence transversalement à la direction de transport (66) et est de préférence conçu en tant que lame fixe, en tant que couteau à ruban ou en tant que couteau rotatif.

6. Procédé selon l'une ou plusieurs des revendications précédentes, dans lequel la surface de travail (30) présente au moins une, de préférence plusieurs, élévation(s) (40) et/ou dépression(s).

7. Procédé selon l'une ou plusieurs des revendications précédentes, dans lequel le substrat (11) est un stratifié (10) adapté à des fins médicales, dans lequel le stratifié (10) comprend au moins une couche poreuse (12), de préférence qui comprend un textile, en particulier une nappe en fibres et/ou un tissu de fibres et/ou une mousse polymère, ainsi qu'une couche de contact avec la plaie (14) qui se distingue de la couche poreuse (12).

8. Procédé selon la revendication précédente, dans lequel la couche poreuse (12) comprend une mousse polymère qui comprend à son tour un polyéther, un polyester, un polyuréthane, un copolymère polyuréthane-polyéther, un acétate de polyvinyle, un alcool polyvinylique, un collagène, un chitosane, une polyoléfine, une silicone ou un mélange d'au moins deux de ces composants.

9. Procédé selon l'une ou plusieurs des deux revendications précédentes, dans lequel la couche de contact avec la plaie (14) comprend une pluralité d'ouvertures (24) qui sont réparties sur la surface et qui, de préférence, s'étendent de la première face extérieure (26) à la couche poreuse (12), ou une structure de type filet.

10. Procédé selon l'une ou plusieurs des 3 revendications précédentes, dans lequel la couche de contact avec la plaie (14) comprend un onguent, une silicone, un hydrogel et/ou un hydrocolloïde.

11. Procédé selon l'une ou plusieurs des 4 revendications précédentes, dans lequel la couche de contact avec la plaie comprend un hydrogel qui comprend à son tour du polyacrylate, du polyuréthane et/ou un copolymère polyuréthane-polyurée.

12. Procédé selon l'une ou plusieurs des revendications précédentes, dans lequel, après avoir coupé les bords du substrat (11) et/ou divisé le substrat (11) selon l'étape vi), une couche de support (90) est appliquée sur la deuxième face extérieure (28), de préférence dans lequel la couche de support (90) présente un revêtement adhésif (88) et la couche de support (90) dépasse le substrat (11) au moins par zones, de préférence sur toute son extension de surface, de telle sorte que le substrat (11) présente un bord adhésif (92) .

13. Procédé selon l'une ou plusieurs des revendications précédentes, dans lequel la première face extérieure (26) du substrat (11) est formée par une doublure détachable ou dans lequel une doublure détachable est appliquée sur la première face extérieure (26), de préférence sur la couche de contact avec la plaie (14), de préférence après avoir coupé les bords du substrat (11) et/ou divisé le substrat (11) selon l'étape vi) ou avant de placer le substrat (11) sur la surface de travail (30) selon l'étape ii).
